# EUROPÄISCHE PATENTSCHRIFT

(11) **EP 2 559 446 B1**
(45) Veröffentlichungstag und Bekanntmachung des Hinweises auf die Patenterteilung: **13.04.2016**
(21) Anmeldenummer: 12005505.8
(22) Anmeldetag: 27.07.2012
(51) Int. Cl.: A61M 1/00, A61F 9/007

(54) **Ophthalmologische Kassette**
Ophthalmologic cassette
Cassette ophtalmologique

(30) Priorität: 19.08.2011 DE 202011104787 U
(43) Veröffentlichungstag der Anmeldung: 20.02.2013
(73) Patentinhaber: EOS GmbH, 52249 Eschweiler (DE)
(72) Erfinder: Klomp, Manfred, 4191 AA Geldermalsen (NL)
(74) Vertreter: Grünecker Patent- und Rechtsanwälte PartG mbB

(56) Entgegenhaltungen:
- US-A- 5 125 891
- US-A- 5 454 783
- US-A1- 2008 015 515
- US-A1- 2008 114 312
- US-A1- 2010 249 693

## Beschreibung

Die vorliegende Erfindung betrifft eine ophthalmologische Kassette zum entfernbaren Anordnen in einer Aufnahme eines ophthalmologischen Geräts.

In der Augenheilkunde ist zur Behandlung von Augenkrankheiten, insbesondere zur Behandlung des grauen Stars, die Phakoemulsifikation die gängige Technik. Bei der Phakoemulsifikation führt der Chirurg eine vibrierende Nadel in das Auge ein, um damit die Augenlinse in kleine Trümmerstücke zu zerkleinern. Zusammen mit Augenflüssigkeit werden die Trümmerstücke als Aspirationsflüssigkeit vornehmlich durch dasselbe vom Chirurg benutzte Handstück abgesaugt. Die abgesaugte Aspirationsflüssigkeit wird in einer vakuumdichten Kassette aufgefangen, die mit einer Vakuumpumpe verbunden ist. Damit das Auge während des Absaugens der Aspirationsflüssigkeit formstabil bleibt und nicht in sich zusammenklafft, wird in das Auge eine Infusionsflüssigkeit eingeleitet, welche im Wesentlichen der natürlichen Augenflüssigkeit entspricht.

Zum Durchführen der Phakoemulsifikation, insbesondere zum Aufnehmen von Absorptionsflüssigkeit, werden in der Praxis verschiedene kassettenförmige Fluidsammelbehälter verwendet. Um am Handstück des Chirurgen eine Saugkraft zu erwirken, wird in der Kassette durch eine fluidtechnisch verbundene Pumpe ein Vakuum gebildet.

Eine herkömmliche Vakuumkassette, die mit einer Vakuumpumpe eines ophthalmologischen Geräts verbindbar ist, ist durch die EP 2 015 796 B1 offenbart. Die Kassette, die zur Aufnahme von Aspirationsflüssigkeit ausgebildet ist, ist zwischen Wandkanten des ophthalmologischen Geräts einschwenkbar angeordnet. Nach dem Einschwenken ist die Kassette mit einer im Gerät befindlichen Pumpe verbindbar. Zum Einschwenken der Kassette sind in einem unteren Bereich vorstehende Bolzen vorgesehen, welche in Ausnehmungen in den Wandkanten des Geräts einführbar sind. Zur Verriegelung der einschwenkbaren Kassette am Gerät ist am Gerät ein Einrasthaken vorgesehen, der in einer Kerbe des oberen Bereichs der Kassette einhakt, um diese zu sichern.

Zum Absaugen der Aspirationsflüssigkeit sowie zum Steuern des Infusionsflusses ist es wichtig, dass der Chirurg mittels Ansteuerung der dafür vorgesehenen Fluidleitungen den Durchfluß einstellen kann. Beispielsweise zeigt die DE 601 07 451 T2 eine ophthalmologische Kassette, wobei an der Rückseite der Kassette Fluidleitungen zum Leiten von Aspirationsflüssigkeit und Infusionsflüssigkeit vorhanden sind. Um in den Fluidleitungen einen Durchfluß zu regeln, sind Ventilelemente vorgesehen.

Alternativ dazu offenbart die US 2007/0287959 A1 eine ophthalmologische Kassette, die Fluidleitungen auf ihrem Deckel aufweist. Die Kassette ist mit einem ophthalmologischen Gerät verbindbar, welches Stossventile umfasst, um die Fluidleitungen auf dem Deckel der Kassette anzusteuern.

US 5,125,891 A offenbart eine ophthalmologische Station mit einer Aufnahme, in welcher eine ophthalmologische Kassette aufnehmbar ist. Die ophthalmologische Kassette kann durch einen Hebel in die Aufnahme gedrückt werden, wobei sie unten an den Seitenwänden Ausnehmungen aufweist, welche auf Halteklammern der Station einrasten. Im gekoppelten Zustand ragt ein Pumpenkopf der ophthalmologischen Station in eine ovale Öffnung der ophthalmologischen Kassette hinein und wird dabei einem Schlauch zugeordnet, welcher im Inneren der Kassette geführt wird. Nachteilig daran ist, dass der Schlauch manchmal beim Einschwenken der Kassette nicht positionsgenau zwischen den Pumpenkopf und die Schlauchführung gerät, was zu Betriebsstörungen der Kassette führen kann sowie eine Herausnahme und ein erneutes Einsetzen der Kassette erlangt.

Der vorliegenden Erfindung liegt die Aufgabe zugrunde, eine ophthalmologische Kassette mittels einfachen, konstruktiven technischen Mitteln dahingehend zu verbessern, dass deren Fluidleitungen zielsicher, funktionell und einfach Steuerelementen eines ophthalmologischen Geräts zugeordnet werden können.

Insbesondere liegt der Erfindung die Aufgabe zugrunde, eine ophthalmologische Kassette mittels einfachen, konstruktiven technischen Mitteln zu schaffen, wobei eine in mancherlei Hinsicht verbesserte Anordnung der vorhandenen Fluidleitungen vorliegt.

Diese Aufgabe wird gelöst mit einer ophthalmologischen Kassette gemäß dem Anspruch 1. Verbesserte Weiterbildungen der Erfindung sind Gegenstand der Unteransprüche.

Die Erfindung betrifft eine ophthalmologische Kassette zur entfernbaren Anordnung in einer Aufnahme eines ophthalmologischen Geräts. Die Kassette eignet sich insbesondere zum Auffangen von Aspirationsflüssigkeit, die beispielsweise während eines chirurgischen Eingriffs am Auge abgesaugt wird. An der Kassette sind Fluidleitungen vorhanden, die mittels am ophthalmologischen Gerät vorgesehener Steuerelemente bedienbar sind. Die Kassette umfasst ein Gehäuse mit einer Vorderwand, einer Rückwand, einem Deckel, einem Boden sowie mit Seitenwänden, wobei vornehmlich am Boden ein Lager für eine schwenkbare Anordnung der Kassette am ophthalmologischen Gerät vorhanden ist.

Gemäß der Erfindung befindet sich an einer der Seitenwände des Gehäuses ein Träger für die Fluidleitungen, der nach dem Einschwenken der Kassette in das Gerät die Steuerelemente den Fluidleitungen funktionell zuordnet. Die Kassette kann vom Benutzer auf einfache Art und Weise in das ophthalmologische Gerät eingeschwenkt werden, wobei gleichzeitig eine funktionelle Zuordnung der Fluidleitungen mit den dafür vorgesehenen Steuerelementen stattfindet.

Vorzugsweise weist das Lager mindestens eine Lagerschale mit einem Führungsbereich auf. Die Lagerschale sorgt für eine solide Lagerung der Kassette und erleichtert es dem Benutzer die Kassette ordnungsgemäß mit dem Gerät zu koppeln. Außerdem erleichtert es die Lagerschale dem Benutzer, die Kassette am Gerät anzuordnen, wobei insbesondere das Einschwenken der Kassette stabil und einfach zu handhaben ist. Der Führungsbereich der Lagerschale bewirkt, dass der Kassette beim Einschwenken eine vorbestimmte Einschwenkrichtung gegeben wird. Dadurch ist es möglich, die Kassette zielgerichtet in das Gerät zu schwenken, wodurch es zu einer sicheren, funktionellen und sterilen Anordnung sowie Ausrichtung der Kassette am Gerät kommt.

In einer weiteren Ausführungsform ist der Führungsbereich zur Kopplung an mindestens einem, zumindest teilweise rotationssymmetrischen Lagerkörper ausgebildet. Auf einem solchen Lagerkörper ist das Einschwenken der Kassette besonders handlich durchzuführen, weil er eine ausreichende Abwälzfläche umfasst, auf der die Lagerschale stabil aufsitzen kann und ohne Verwackeln in das Gerät schwenkbar ist. Mit einem solchen Lagerkörper ist eine gleichmäßige Schwenkbewegung der Kassette in aber auch aus dem Gerät möglich. Vorzugsweise ist der Führungsbereich zur Kopplung an einem zylindrischen oder konischen oder birnenförmigen oder ähnlichen rotationssymmetrischen Körper ausgebildet. Ein Führungsbereich dieser Form ist insbesondere dann hilfreich, um Kassette eindeutig in eine vorgegebene Schwenkrichtung zu versetzen. Ein derartiger Führungskörper kann dem Benutzer vor allem behilflich sein, wenn die Kassette beim Andocken an den Führungsbereich nicht sofort optimal zu diesem ausgerichtet ist. Es hat sich nämlich gezeigt, dass die Kassette selbst bei einem unbestimmten Aufsetzen der Lagerschale auf einem der oben benannten Lagerkörper auf einfache und ergonomische Art und Weise in die vorbestimmte Schwenkrichtung ausrichtbar ist. Beispielsweise wäre es möglich, die Kassette zunächst schräg auf dem Lagerkörper anzusetzen, wobei sich die Kassette dann leicht in die vorbestimmte Schwenkrichtung kippen lässt. Somit ist es möglich, die Kassette auf einfache Art und Weise und vor allem ergonomisch in eine ordnungsgemäße Ausgangsposition zu bringen, um sie dann in der vorbestimmten Einschwenkrichtung in das Gerät einzuschwenken.

In einer verbesserten Ausführungsform sind im Führungsbereich der Lagerschale vornehmlich in der vorbestimmten Schwenkrichtung der Kassette angeordnete Ausrichtungsrippen vorhanden. Diese helfen dem Benutzer, die Kassette in Schwenkrichtung auszurichten und diese zu stabilisieren. Insbesondere vorteilhaft ist es, wenn die Ausrichtungsrippen zum Erreichen der vorbestimmten Schwenkrichtung an den Lagerkörper angepasst sind. Durch die angepasste Form der Ausrichtungsrippen ist es möglich, dass diese beim Koppeln der Kassette automatisch an die Form des oder der Lagerkörper herangeführt werden, wodurch die Einschwenkrichtung einfach und zielsicher erreichbar ist. Die Ausrichtungsrippen sorgen auch dafür, dass sich die Kassette relativ zum Lagerkörper nicht während des Einschwenkens seitlich verschiebt. Folglich ist eine besonders präzise Anordnung der Kassette im Gerät möglich.

Besonders hilfreich und ergonomisch für den Benutzer ist es, wenn die Lagerschale die Form einer Rinne aufweist. Dadurch kann nicht nur das Einschwenken der Kassette erleichtert werden, vielmehr führt die Form einer Rille ebenfalls zu einer verbesserten Stabilität der Kassette. Schließlich kann der Benutzer die Kassette mit der Rinne einfach im Lager anordnen.

Vorzugsweise ist die Lagerschale einteilig am Boden der Kassette ausgebildet, wodurch es insbesondere im Bereich der Lagerung zu einer verbesserten Robustheit der Kassette kommt. Durch die einteilige Ausbildung werden Lagerkomponenten reduziert und Herstellkosten optimiert. Es kann auch vorteilhaft sein, wenn sich die Lagerschale mindestens abschnittsweise entlang des Bodens erstreckt. Dies ermöglicht, dass die Kassette gut in das Lager ankoppelbar ist. Ebenfalls kann dadurch verhindert werden, dass die Kassette insbesondere während des Einschwenkens seitlich in der Lagerung kippt.

Besonders stabil ist das Lager ausgebildet, wenn sich die Lagerschale im Wesentlichen entlang der gesamten Breite des Bodens erstreckt. Damit ist es insbesondere auch möglich die Kassette ortsfest im Gerät so halten, selbst wenn es zu Druckschwankungen innerhalb der Kassette kommt oder die Fluidleitungen angesteuert werden. Um eine besonders kompakte Form für das Lager zu erhalten, ist es möglich, die Lagerschale vorzugsweise teilweise im Boden der Kassette versenkt einzuformen. Für eine stabile Lagerung der Kassette, insbesondere um ein seitliches Abkippen der Kassette zu vermeiden, ist es auch sinnvoll, wenn ein erstes und ein zweites Ende der Lagerschale über die Breite des Bodens hinausragt. Damit kann insbesondere die Seitenstabilität der Lagerung verbessert werden. Damit die Lagerschale am vorgesehenen Lagerkörper ergonomisch andockbar ist, ist es vorteilhaft, wenn die Lagerschale am ersten und/oder am zweiten Ende abgerundet ist. Die Abrundung an der Lagerschale kann auch verhindern, dass die Kassette im Bereich des Lagers im Gerät verkantet.

Um die Kassette im Gerät zu befestigen, umfasst sie vornehmlich am Deckel eine Verriegelung, durch die sie nach dem Einschwenken am Gerät lösbar gesichert werden kann. Vorzugsweise ist die Verriegelung als Feder ausgebildet. Folglich ist die Verriegelung zwischen mindestens zwei Positionen bewegbar, nämlich einer geöffneten und einer geschlossenen Position. Die als Feder ausgeführte Verriegelung ist einfach und schnell zu schließen beziehungsweise zu öffnen. Vorzugsweise federt die Verriegelung selbsttätig in die geschlossene Position, sodass dem Benutzer eine einfache Handhabe vorliegt.

In einer weiteren Ausführungsform der Erfindung ist vorgesehen, dass die Verriegelung die Kassette gegen das Lager vornehmlich federnd spannt. Dies bietet insbesondere den technischen Vorteil, dass die Kassette während einem chirurgischen Eingriff sicher und stabil in der Lagerung haltbar ist und nicht durch Vibrationen aus der Lagerung ausschnappt. Außerdem führt das Einspannen der Kassette gegen das Lager dazu, dass thermische Verformungen der Kassette, beziehungsweise des Geräts ausgleichbar sind. Vorstellbar ist auch, dass Herstellungstoleranzen in der Bemaßung der Kassette ausgleichbar sind.

Um die Kassette sicher in der Verriegelung zu halten, umfasst die Verriegelung vorzugsweise eine Einrastplatte, die insbesondere im Wesentlichen parallel zum Deckel angeordnet ist. Die Einrastplatte ist vornehmlich so bemaßt, dass jedenfalls ein Finger des Benutzers, insbesondere der Daumen, auf ihre Oberfläche drücken kann, um die Verriegelung zu öffnen. Außerdem ist die Bemaßung so gewählt, dass die Einrastplatte selbst dann direkt vom Benutzer betätigbar ist, wenn die Kassette im Gerät angeordnet ist. Deshalb kann es vorteilhaft sein, die Einrastplatte so auszubilden, dass sie über die Vorderwand der Kassette hinausragt.

Um die Einrastplatte federnd am Gehäuse der Kassette zu lagern, so dass sie beweglich am Deckel angeordnet ist, umfasst diese einen gekrümmten Bereich, der die Einrastplatte mit dem Deckel der Kassette verbindet. Alternativ dazu kann es sich auch um einen rechteckig ausgebildeten Bereich handeln. Der gekrümmte Bereich ist vorzugsweise mittig an einer Kante ausgebildet, die den Deckel und die Rückwand der Kassette verbindet. Der gekrümmte Bereich durchläuft vorzugsweise eine 90° Biegung, sodass er die Einrastplatte über dem Deckel im Wesentlichen parallel anordnet und diese so ausrichtet, dass sie nach vorn gerichtet über den Deckel ragt.

Zum Befestigen der Kassette im Gerät rastet die Einrastplatte am Gerät ein. Dazu umfasst die Einrastplatte eine Verschlusskante, die sich auf der Einrastplatte entlang deren Breite symmetrisch zur Schwenkrichtung erstreckt, vornehmlich jedoch quer zur vorbestimmten Schwenkrichtung geformt ist, wobei die Verschlusskante vorzugsweise näher am gekrümmten Bereich als an einer dem Benutzer zugewandten Kante der Einrastplatte verläuft. Vorzugsweise ist die Verschlußkante so geformt, dass sie zum Gerät hin relativ zur Oberfläche der Einrastplatte eine Schräge, also Rampe, aufweist, um das Einrasten zu erleichtern. Andererseits ist die Einrastkante im Wesentlichen orthogonal zur Oberfläche der Einrastplatte geformt, um die Einrastfunktion zu gewährleisten.

Vorzugsweise kontaktiert die Verschlusskante der Einrastplatte eine Verriegelungsleiste des Geräts, wenn die Kassette am Gerät verriegelt ist. Dadurch kann verhindert werden, dass die Kassette während eines chirurgischen Eingriffs in der Lagerung wackelt, geschweige denn aus dieser rausfällt.

Der Benutzer kann die Kassette lediglich durch das Einschwenken in das Gerät verriegeln, wobei an einer vorbestimmten Endlage der Kassette dessen Verrieglung am Gerät einrastet. Vorzugsweise kann der Benutzer allein durch Drücken der Oberfläche der Einrastplatte die Verriegelung lösen, um die Kassette aus dem Gerät zu entnehmen. Dadurch wird der Mechanismus der Verriegelung vereinfacht, wobei die zur Verrieglung vorgesehen Mittel kostengünstig herstellbar sind.

In einer weiteren vorteilhaften Ausführungsform der Erfindung ist die Verriegelung, insbesondere die Einrastplatte dazu ausgebildet, ein Geräusch zu erzeugen, wenn sie am Gerät einrastet. Vornehmlich ist dabei ein eindeutiges Klickgeräusch vom Benutzer wahrnehmbar. Dies bietet den technischen Vorteil, dass der Bediener sofort über ein ordnungsgemäßes Einrasten beziehungsweise über eine ordnungsgemäße Verriegelung der Kassette informiert wird. Das Klickgeräusch kann dadurch zustande kommen, dass die Einrastplatte, nachdem die Verriegelungsleiste des Geräts über die Rampe der Verschlusskante gedrückt wurde, schlagartig nach oben zurückfedert und die Verriegelungsleiste des Geräts hinter der Rampe auf der Oberfläche der Einrastplatte aufschlägt. Dies bietet insbesondere eine kostengünstige Lösung zur Wahrnehmung einer einwandfreien Verriegelung der Kassette im Gerät, bei der auf teuere elektronische Sensorik verzichtet werden kann.

Vorzugsweise sind in der Rückwand der Kassette Öffnungen für eine dem Gerät zugeordnete Pumpe, für eine Belüftung sowie für eine Aspirationsleitung vorhanden. Die Ausbildung der Öffnungen an der Rückseite der Kassette verbessert die Sterilität der Kassette während eines chirurgischen Eingriffs. Außerdem kann zusätzlich verhindert werden, dass ungewollte Berührungen beziehungsweise Kontakte mit den Öffnungen zustande kommen. Schließlich können insbesondere die Öffnungen für die Belüftung sowie für die Vakuumpumpe funktionell im Gerät durch das Einschwenken der Kassette zugeordnet werden.

In einer weiteren Ausführungsform der Erfindung ist nach dem Einschwenken der Kassette in das Gerät eine vakuumdichte Fluidverbindung zwischen der Pumpe und der dafür vorgesehenen Öffnung herstellbar. Durch diese Fluidverbindung ist beabsichtigt, dass die Pumpe durch das Ansaugen von Luft ein Vakuum in der vakuumdichten Kassette erzeugt. Die durch das Vakuum entstehende Sogwirkung ist auf die Öffnung der Aspirationsflüssigkeit übertragbar, um durch diese Aspirationsflüssigkeit in die Kassette zu saugen. Weil die Fluidverbindung zwischen der Pumpe und der dafür ausgebildeten Öffnung lediglich durch das Einschwenken der Kassette in das Gerät herstellbar ist, müssen keine weiteren Handgriffe vom Bedienpersonal unternommen werden. Dies erleichtert die Inbetriebnahme der Kassette vor Ort und verbessert gleichzeitig die Sterilität des Eingriffs.

Vorzugsweise ist die Öffnung für das Eintreten der Aspirationsflüssigkeit derart gestaltet beziehungsweise die Pumpe für das Erzeugen des Vakuums in der Kassette derart geregelt, dass die Aspirationsflüssigkeit in die Kassette so eintritt, dass keine durchgehende Fluidverbindung zwischen der Aspirationsflüssigkeit an der Eintrittsöffnung und der Aspirationsflüssigkeit am Boden der Kassette besteht. Dies ist wichtig, um die Sterilität der Aspirationsleitung zu verbessern. Folglich ich es möglich zu verhindern, dass kontaminierte Aspirationsflüssigkeit an die Eintrittsöffnung der Aspirationsflüssigkeit gelangt.

Vorteilhaft ist es auch, wenn nach dem Einschwenken der Kassette in das Gerät eine Fluidverbindung zwischen der Belüftung und der dafür vorgesehenen Öffnung herstellbar ist. Diese Fluidverbindung installiert die Zufuhr von Luft. Ein Öffnen und Schließen der für die Belüftung vorgesehenen Öffnung ist vorzugsweise durch ein Belüftungssteuerelement durchführbar. Das Belüftungssteuerelement ist in der für die Kassette vorgesehenen Aufnahme relativ zur Rückseite der Kassette angeordnet. Vorzugsweise ist das Belüftungssteuerelement funktionell mit einem Fußschalter verbunden, wobei es durch ein Entlasten des Fußschalters die Belüftungsöffnung öffnet, so dass die Kassette belüftet wird. Bei einer erneuten Betätigung des Fußschalters schließt das Belüftungssteuerelement die Belüftungsöffnung, wodurch es zum erneuten Aufbau eines Vakuums in der Kassette kommt. In einer weiteren Ausführungsform ist vorgesehen, dass während der geöffneten Belüftung die bereits in der Kassette angesammelte Flüssigkeit in einen mit der Vorderwand der Kassette verbundenen Fluidsammelbeutel abläuft. Dadurch kann verhindert werden, dass sich während einer Operation zu viel Flüssigkeit in der Kassette ansammelt.

Eine besonders vorteilhafte Anordnung der Fluidleitungen ist gegeben, wenn der Träger für die Fluidleitungen eine Ausrichtplatte umfasst, die an einer der Seitenwände ausgebildet ist. Auf der Ausrichtplatte können die Fluidleitungen gezielt so angeordnet werden, dass sie den Steuerelementen funktionell und zuverlässig zuteilbar sind, wobei die Ausrichtplatte ebenso ein robustes Widerlager definiert, um die Fluidverbindungen darauf zu betätigen.

In einer besonderen Ausführungsform der Erfindung weist die Ausrichtplatte zur vorbestimmten Schwenkrichtung eine Neigung zwischen 2 und 7 Grad auf. Dies bedeutet, dass sie vorzugsweise nicht exakt orthogonal zur Rückwand ausgerichtet ist, sondern relativ zur Rückwand einen größeren Winkel, bis ungefähr 97 Grad einschließt. Dies bietet den technischen Vorteil, dass die Ausrichtplatte beim Einschwenken der Kassette in das Gerät in eine gewisse Vorspannung geht, wodurch die Fluidleitungen besonders präzise den Steuerelementen zugeordnet werden können. Außerdem kann durch die Vorspannung der Ausrichtplatte dieselbe eine den Steuerelementen entgegenwirkende Kraft aufbringen, die das Schließen der Fluidleitungen erleichtert, wenn die Steuerelemente darauf wirken.

Die zuvor beschriebene, zur Schwenkrichtung leicht geneigte Ausrichtplatte bietet ebenfalls den technischen Vorteil einer Einschwenkhilfe, weil während des Verlaufs des Einschwenkens der Kassette in die dafür vorgesehene Aufnahme des Geräts zunächst eine breitere Einschwenköffnung für die Ausrichtplatte zur Verfügung steht, wobei die Ausrichtplatte nach erfolgtem Einschwenken der Kassette in die dafür vorgesehene Aufnahme in ihrer Endposition kontaktschlüssig gegen die Aufnahme anliegt. Folglich kann verhindert werden, dass beim Einschwenken der Kassette die Ausrichtplatte an der Aufnahme hängen bleibt, wobei gleichzeitig ermöglicht wird, dass nach vollständigem Einschwenken der Kassette ein Kraftschluss zwischen der Ausrichtplatte und der ausgebildeten Aufnahme entsteht, so dass eine Betätigung des Trägers, insbesondere der Fluidleitungen, verbessert wird.

Um die Fluidleitungen stabil auf der Ausrichtplatte zu führen, sind auf der Ausrichtplatte Halter vorgesehen, durch die die Fluidleitungen funktionstauglich relativ zu den Steuerelementen positionierbar sind. Die Halter verhindern, dass sich die Fluidleitungen auf der Ausrichtplatte verschieben, wenn die Steuerelemente auf sie wirken. Vorteilhaft ist es, wenn die Halter insbesondere senkrecht auf der Ausrichtplatte stehen, weil dies einfach herstellbar ist. Dabei können die Halter entweder eine zylindrische nadelartige Form oder in Form einer Platte ausgebildet sein. Möglich ist es auch, dass lediglich Platten oder nadelartige Formen für die Halter vorhanden sind, um die Fluidleitungen auf der Ausrichtplatte zu halten und zu lenken. Nadelförmige Halter sind insbesondere dann von Vorteil, wenn die Fluidleitungen eine Kurve durchlaufen, während plattenartige Halter vorzugsweise zum Führen und Fixieren der Fluidleitungen entlang eines geraden Abschnitts vorteilhaft sind. Vorzugsweise werden die plattenartigen Halter dazu verwendet, um Verbinder der Fluidleitungen auf der Ausrichtplatte zu positionieren. Dabei sind die plattenartigen Halter vorzugsweise an der Stelle auf der Ausrichtplatte vorhanden, wo Endabschnitte der Fluidleitungen mit den Verbindern verbunden sind. Die aus einem härteren Material als die elastischen Fluidleitungen hergestellten Schlauchverbinder können dadurch verhindern, dass die plattenartigen Halter die Fluidleitungen abdrücken. Um der Ausrichtplatte eine biege- und torsionssteife Struktur zu geben, ist es sinnvoll, wenn am Rand der Ausrichtplatte mindestens teilweise eine Umrandung vorhanden ist. Außerdem bietet die Umrandung einen Schutz für die Fluidleitungen, die auf der Ausrichtplatte positioniert sind. Die Ausrichtplatte kann zusätzlich mit mindestens einer Einrastkerbe ausgestattet sein, um beispielsweise eine Abdeckung darin zu befestigen.

Vorzugsweise ist die Ausrichtplatte einteilig mit einer hinteren Kante der Seitenwand verbunden. Außerdem kann es vorteilhaft sein, auf der den Fluidleitungen entgegengesetzten Seite der Ausrichtplatte Rippen vorzusehen, die von der hinteren Kante der Seitenwand entlang der Ausrichtplatte zu deren abstehenden Ende verlaufen. Die Rippen geben der Ausrichtplatte zusätzlich Stabilität. In einer weiteren Ausführungsform ist vorgesehen, dass die Rippen jeweils direkt hinter den Stempelpositionen an der Ausrichtplatte vorgesehen sind, um den Kräften, die durch die Steuerelemente beim Schließen der Fluidleitungen auf die Ausrichtplatte wirken, entgegen zu wirken, als auch die Kräfte an das kraftschlüssig anliegende Aufnahmemodul weiterzugeben. Dadurch kann insbesondere vermieden werden, dass die Ausrichtplatte vom Gehäuse der Kassette abbricht. Um die Führung der Fluidleitungen zusätzlich zu verbessern ist es sinnvoll, wenn an der Seitenwand, die die Ausrichtplatte trägt, mindestens eine Führungsleiste ausgebildet ist. Die Führungsleiste ermöglicht es auch, dass die Fluidleitungen in einer vorgegeben Richtung von der Kassette weglaufen. Vorzugsweise ist die Führungsleiste senkrecht auf der Seitenwand ausgebildet, wobei zur sicheren Halterung der Fluidleitungen Öffnungen in der Führungsleiste vorhanden sind, in welchen die Fluidleitungen eingeklemmt werden können. Für eine besonders stabile Ausführung der Führungsleiste ist es vorgesehen, dass an der Führungsleiste mindestens eine Stützstrebe ausgebildet ist, durch welche die Führungsleiste stabil an der Seitenwand befestigt ist. Vorzugsweise kann die Führungsleiste an eine Kante der Vorderwand einteilig angeformt sein, sodass sie orthogonal zur Seitenwand ausgerichtet ist.

Um die Stabilität des Trägers zu verbessern und um die Fluidleitungen vor äußeren Einflüssen zu schützen, umfasst der Träger eine Abdeckung, die mit der Ausrichtplatte verbindbar ist. Die Ausrichtplatte bildet zusammen mit der Abdeckung ein Trägergehäuse für die Fluidleitungen. Für eine bessere Stabilität der Abdeckung kann vorgesehen sein, dass sie mindestens teilweise eine Abdeckungsumrandung umfasst. Diese kann beispielsweise so ausgebildet sein, dass sie die Umrandung der Ausrichtplatte im Wesentlichen so ergänzt, dass eine in sich geschlossene Umrandung für das Trägergehäuse vorliegt. Ebenfalls ist es vorstellbar, dass mindestens ein Einrastglied vornehmlich am Rand der Abdeckung vorhanden ist, um dieses in der Einrastkerbe der Ausrichtplatte zu befestigen. Das Einrastglied kann allerdings auch an anderer Stelle der Abdeckung angeordnet sein. Nützlich wäre es auch, wenn Führungsabschnitte sowohl auf der Abdeckung als auch auf der Ausrichtplatte vorhanden wären, um das Einrastglied mit der Einrastkerbe auszurichten, sodass die Abdeckung leicht mit der Ausrichtplatte verbindbar ist. Damit eine Fluidleitung für Aspirationsflüssigkeit problemlos aus dem Trägergehäuse zu der im Gehäuse vorgesehnen Aspirationsöffnung führbar ist, ist es vorteilhaft, wenn in der Abdeckungsumrandung mindestens eine Umrandungsöffnung ausgebildet ist. Vorteilhaft ist auch, wenn in der Abdeckung Betätigungsöffnungen vorhanden sind, durch welche die Steuerelementte schiebbar sind, um den Fluidfluss zu beeinflussen.

Zum Einsatz die Kassette bei einer Augenoperation beispielsweise zur Behandlung des grauen Stars sind als Fluidleitungen vorzugsweise eine Aspirationsleitung für ein Aspirationsfluid, eine Infusionsleitung für ein Infusionsfluid und eine Backflushleitung für den Fall einer Okklusion in der Aspirationsleitung vorhanden. Das Aspirationsfluid enthält zerkleinerte Trümmerstücke der Augenlinse sowie Augenflüssigkeit und wird vom Auge in die vakuumdichte Kassette abgesaugt. Die Infusionsflüssigkeit wird durch die Infusionsleitung dem Auge bereitgestellt, damit es stabil bleibt und nicht in sich zusammenklafft. Die Backflushleitung kann vom Chirurg geöffnet werden, wodurch Infusionsflüssigkeit durch die Backflushleitung in die Aspirationsleitung geleitet wird. Dadurch kann reaktionsschnell das Absaugen von Aspirationsfluid aus dem Auge unterbrochen werden. Ein Einsatz der Backflushleitung ist vor allem dann wichtig, wenn die Aspirationsleitung beispielsweise durch ein Stück der Linse verstopft, womit der Unterdruck schlagartig in der Kassette ansteigt. Wenn sich dann das Stück der Linse lösen sollte, kann es zu einem riskanten abrupten Absaugen von Aspirationsflüssigkeit kommen, wodurch schlimmstenfalls bleibende Schäden am Auge eintreten können.

Um zu gewährleisten, dass das abgesaugte Aspirationsfluid mit den Trümmerstücken der Linse in der Kassette landet, ist die Aspirationsleitung mit der dafür vorgesehenen Öffnung der Kassette verbunden. Diese Verbindung ist vakuumdicht und steril.

In einer Ausführungsform verbindet ein T-förmiger Verbinder, der im Trägergehäuse angeordnet ist, zwei Enden der Infusionsleitung sowie ein Ende der Backflushleitung. Dadurch ist es möglich Infusionsflüssigkeit in die Backflushleitung einzuleiten, um den Backflush, also Rückstoß, in der Aspirationsleitung zu erhalten. Außerdem kann im Träger ein Y-förmiger Verbinder vorhanden sein, um ein zweites Ende der Backflushleitung mit der Aspirationsleitung zu verbinden, wobei die anderen zwei Enden des Y-förmigen Verbinders Abschnitte der Aspirationsleitung verbinden. Schließlich kann ein vornehmlich L-förmiger Verbinder vorgesehen sein, um Abschnitte der Infusionsleitung zu verbinden. Der T-, Y- und L-förmige Verbinder sorgen auch dafür, dass die Fluidleitungen im Träger kompakt in einer bestimmten Richtung angeordnet sind.

Damit es möglich ist, den Fluss des Aspirationsfluids, des Infusionsfluids sowie die Ansteuerung der Backflushleitung zu regeln, ist es vorteilhaft, wenn die Aspirationsleitung, die Infusionsleitung sowie die Backflushleitung durch jeweils ein zugeordnetes Steuerelement betätigbar sind. Die Steuerelemente sind vorzugsweise als pneumatische Zylinder ausgebildet, die jeweils einen ausfahrbaren Kolben umfassen, welcher durch Ansteuerung der Steuerelemente zielgerichtet auf die zugeordnete Fluidleitung drückt, um darin einen Fluidfluß zu unterbinden. Durch Einfahren des Kolbens kann der Fluidfluß wieder ermöglicht werden. Die Steuerelemente sind unabhängig voneinander betätigbar, wobei auch ein gleichzeitiges Ausfahren und/oder Einfahren der Steuerelemente vorstellbar ist.

Beispielsweise kann die Aspirationsleitung durch das vorgesehene Steuerelement geschlossen werden, um den Aspirationsfluß zu verhindern, während gleichzeitig die Belüftung, ebenfalls durch ein Steuerelement, vornehmlich einen Pneumatikzylinder, geöffnet wird, um den Unterdruck in der Kassette abzubauen. Ebenfalls ist es möglich im Falle einer Okklusion der Aspirationsleitung, die Backflushleitung zu öffnen, damit dem angesaugten Aspirationsfluss ein Infusionsstoß entgegenwirkt, während gleichzeitig das Steuerelement für die Infusionsleitung ausfährt, um den Infusionsfluß zu stoppen.

In einer weiteren vorteilhaften Ausführungsform der Erfindung sind die Fluidleitungen im Wesentlichen orthogonal zur Schwenkrichtung der Kassette durch die Steuerelemente betätigbar. Anders gesagt in orthogonaler Richtung zu den Seitenwänden der Kassette. Dabei wirken bei der orthogonalen Betätigung der Fluidleitungen durch die Steuerelemente als Widerlager die Ausrichtplatte und/oder die Aufnahme des Geräts. Dadurch ist es möglich, dass die Fluidleitungen durch die Steuerelemente dicht schließbar sind, um einen Fluidfluss zu verhindern. Außerdem ist es durch die orthogonale Betätigung der Fluidleitungen nicht möglich, die Kräfte ausfahrender Steuerelemente in das Lager einzuleiten, wodurch die Kassette möglicherweise aus der Lagerung fällt. Deshalb ergibt sich durch die orthogonale Betätigung ebenfalls keine wesentliche Beanspruchung des Lagers. Ein Herausdrücken der Kassette aus dem Gerät ist folglich nicht möglich.

Um eine Belüftungsfunktion anhand der Belüftung innerhalb des Gehäuses der Kassette zu verbessern, ist es vorgesehen, dass im Inneren des Gehäuses der Kassette an der Rückwand Lüfterrippen geformt sind. Vorzugsweise weisen die Lüfterrippen eine erste und eine zweite Lüftverteilerrippe auf, die zusammen im Wesentlichen einen nach unten konischen Trichter bilden. Insbesondere ist es durch die konische Trichterform möglich, dass ein eingeführter Luftstrom sich gleichmäßig über die Breite der Kassette verteilt, was zur Folge hat, dass der eingesaugte Luftstrom gleichmäßig auf die angesammelte Aspirationsflüssigkeit trifft. Dies führt zu einem kontrollierten Entlüften der Kassette, wobei Fluidschwankungen in der Kassette nicht zu befürchten sind. Besonders nützlich ist es, wenn die Lüfterrippen zusätzlich Dosierrippen aufweisen, die vornehmlich dachartig, schräg gestellt angeordnet sind. Die dachförmige Anordnung stellt sicher, dass für den Fall, wenn doch mal Aspirationsflüssigkeit auf die Dosierkippen gelangen sollte, die Aspirationsflüssigkeit schnell wieder abfließen kann. Die Dosierrippen verhindern, dass der eingesaugte Luftstrom schlagartig und abrupt auf die angesammelte Aspirationsflüssigkeit einwirkt. Vielmehr ermöglichen sie es, dass der Luftstrom langsam und gleichmäßig dosiert einleitbar ist, so dass es zu keiner Schaumbildung kommt.

Um ein hin und her Schwappen der angesammelten Aspirationsflüssigkeit im Inneren des Gehäuses der Kassette zu verhindern, ist es nützlich, wenn Strömungsrippen vornehmlich parallel zu den Seitenwänden der Kassette vorhanden sind. Diese führen zur Beruhigung der Aspirationsflüssigkeit im Gehäuse. Vorteilhaft ist es auch, wenn die Strömungsrippen nicht gänzlich bis zum Boden des Gehäuses weichen, sodass eine gleichmäßige Auffüllung des Gehäuses mit Aspirationsflüssigkeit möglich ist.

Zur Anzeige des Füllstands der Kassette ist vorzugsweise ein Schwimmer zwischen zwei der Strömungsrippen vorhanden. Vornehmlich ist der Schwimmer als Kugel ausgebildet und lässt sich durch das Ansteigen der Aspirationsflüssigkeit in der Kassette nach oben heben. Um zu erkennen, an welcher Position sich der Schwimmer in der Kassette befindet, ist es vorteilhaft, wenn insbesondere an der Rückwand des Gehäuses relativ zum Schwimmer ein Fenster ausgebildet ist. Der Schimmer kann dann durch vorgesehene sensorische Mittel erfasst werden, sobald er sich auf der Höhe des Fensters befindet. Vorzugsweise kann das Erkennen der Schwimmers im Fensterbereich dazu führen, dass dem Benutzer ein Warnsignal widergegeben wird, beispielsweise ein Piepton, durch den der Benutzer zur Entnahme der Kassette aufgefordert werden soll.

Vorzugsweise ist im Inneren des Kassettengehäuses an der Öffnung der Pumpe eine Filterhalterung vorhanden, die einen Filter trägt. Der Filter verhindert, dass Aspirationsflüssigkeit das Gehäuse der Kassette verläßt. Insbesondere vorteilhaft ist es, wenn der Filter lediglich durch Presspassung in der Filterhalterung haltbar ist. Dies erleichtert die Herstellung erheblich, wobei zusätzliche Befestigungsmaterialien, wie beispielsweise Klebmittel, nicht benötigt werden.

Optional sind im Inneren der Kassette an der Vorderwand im Wesentlichen die gleichen Lüfterrippen und Strömungsrippen wie an der Rückwand der Kassette ausgebildet, wobei diese spiegelgleich angeordnet sind. Um dem Gehäuse eine besonders stabile Struktur zu geben, ist in einer weiteren Ausführungsform der Erfindung vorgesehen, dass die Lüfterrippen sowie Strömungsrippen der Rückwand die Lüfterrippen sowie Strömungsrippen der Vorderwand sich bei Sogzustand aufeinander abstützen. Damit die Lüfterrippen nicht aufeinander reiben, ist zwischen ihnen vorzugsweise eine Gummierung als Dichtung vorgesehen.

Für eine benutzerfreundliche Handhabe der Kassette ist auf deren Vorderwand ein Griff vorhanden. Dieser ist insbesondere in der Form eines Henkels ausgebildet, damit der Benutzer die Möglichkeit hat insbesondere mit seinem Zeigefinger in die Öffnung des Griffs einzuhaken, um feinfühlig die Kassette in die Lagerung zu legen, sodass die Kassette in das Gerät einschwenkbar ist.

Vorzugsweise ist der Griff als Lichtleiter ausgebildet. Dazu nimmt der Griff an einem oberen Ende einen Lichtstrahl auf und leitet diesen zu einem unteren Ende weiter. Das am unteren Ende austretende Licht kann von Mitteln zur Detektion des Lichts, welche hinter der Rückwand der Kassette im Gerät vorgesehen sind, detektiert werden, falls eine in der Kassette vorgesehne Schwimmerkugel den Lichtstrahl nicht unterbricht. Der durch den Griff aufgenommene und weitergeleitete Lichtstrahl wird dann unterbrochen, wenn die Schwimmerkugel durch einen vorbestimmten Füllstand der Kassette soweit angehoben wird, dass sie verhindert, dass das Licht die Mittel zur Detektion erreicht.

In einer vorteilhaften Ausführungsform der Kassette ist diese aus transparentem Material ausgebildet, insbesondere Kunststoff. Dadurch kann der Benutzer ins Innere der Kassette sehen, um gegebenenfalls festzustellen, wann diese voll ist.

Anhand der folgenden Zeichnungen werden Ausführungsbeispiele der Erfindung dargestellt. Es zeigen:
- Figur 1: eine Seitenansicht des Geräts mit aufgenommener Kassette,
- Figur 2: eine perspektivische Ansicht der erfindungsgemäßen ophthalmologischen Kassette,
- Figur 3: eine weitere perspektivische Ansicht der Kassette, der Figuren 1 und 2,
- Figur 4: eine Seitenansicht der Kassette, der Figuren 1, 2 und 3,
- Figur 5: die Abdeckung des Trägergehäuses im abgebauten Zustand,
- Figur 6: eine Seitenansicht der Kassette vorstehender Figuren mit Fluidleitungen, nämlich. Aspirationsleitung, Infusionsleitung sowie Backflushleitung,
- Figur 7: die innere Struktur der ophthalmologischen Kassette gemäß der Erfindung,
- Figur 8: eine Seitenansicht auf die für die Kassette vorgesehene Aufnahme,
- Figur 9: einen vertikalen Schnitt durch die Kassette und die Aufnahme des Gehäuses gemäß Linie IX - IX von Figur 8,
- Figur 10: eine Vorderansicht der Kassette in der Aufnahme des Gehäuses,
- Figur 11: eine vertikale Schnittdarstellung der Kassette in der Aufnahme des Geräts gemäß der Linie XI - XI von Figur 10
- Figur 12: eine Vorderansicht der in der Aufnahme befindlichen Kassette mit der Abbildung der Steuerelemente und
- Figur 13: einen horizontalen Schnitt durch die Kassette, und des Trägergehäuses gemäß Linie XIII - XIII der Figur 12.

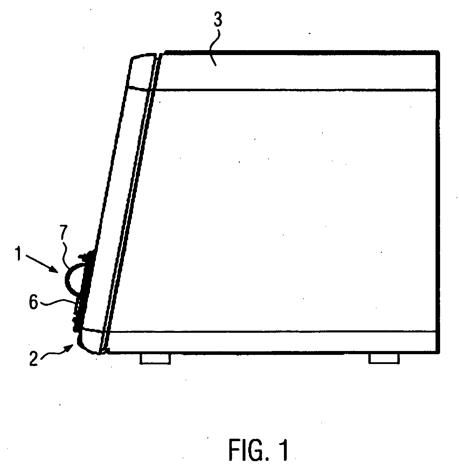

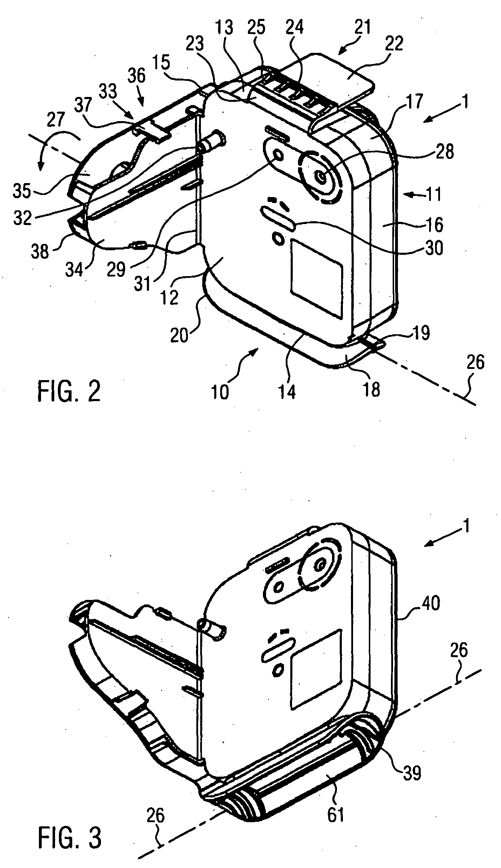

Die Figur 1 zeigt eine ophthalmologische Kassette 1, die in einer Aufnahme 2 eines ophthalmologischen Geräts 3 angeordnet ist. Die Aufnahme 2 ist in einer Vorderseite des ophthalmologischen Geräts 3 ausgebildet. Auf der Vorderseite des Geräts können zusätzliche Anschlüsse vorgesehen sein, wie beispielsweise Steckerpositionen für einen Phako Handgriff, eine Diathermiepinzette und/oder einen pneumatischen Cutter 4. Von der Kassette 1 ist im Wesentlichen eine Vorderwand 6 zu sehen, die sich an in der Ebene der Vorderseite des Geräts 3 erstreckt. Für eine verbesserte Handhabung der Kassette 1 ist auf der Vorderseite 6 ein Griff 7 vorhanden. Die Figur 2 zeigt die Kassette 1 von dem ophthalmologischen Gerät 3 gelöst. Die Kassette 1 umfasst ein Gehäuse 11 mit der Vorderwand 6, einer Rückwand 12, einem Deckel 13, einem Boden 14 sowie Seitenwänden 15, 16. Außerdem sind für das Gehäuse 11 abgerundete Ecken 17 zwischen dem Deckel 13, dem Boden 14 und den Seitenwänden 15, 16 vorgesehen. Am Boden 14 der Kassette 1 für das Lager 10 eine Lagerschale 18 vorgesehen. Die Lagerschale 18 erstreckt sich im Wesentlichen über die gesamte Breite des Bodens 14. Im gezeigten Beispiel ragt sie sogar über die Breite des Bodens 14 hinaus. Die Lagerschale 18 hat die Form einer Rinne, wobei sie an einem ersten und an einem zweiten Ende 19, 20 abgerundet ist. Die Lagerschale 18 ist einteilig mit dem Gehäuse 11 der Kassette 1 ausgebildet und gehört zum Lager 10.

Auf dem Deckel 13 der Kassette 1 ist eine Verriegelung 21 angeordnet. Die Verrieglung 21 umfasst eine Einrastplatte 22, die im Wesentlichen parallel zur Oberfläche des Deckels 13 ausgerichtet ist. Die Einrastplatte 22 ist über einen abgerundeten Bereich 23 mit dem Deckel 13 der Kassette 1 verbunden, welcher gemäß der Figur 2 entlang einer Kante des Deckels 13 ausgebildet ist. Der abgerundete Bereich 23 ermöglicht es, dass die Einrastplatte 22 relativ zur Kassette 1 federnd auf dem Deckel 13 gelagert ist. Sichtbar ist auch, dass die Einrastplatte 22 über die Vorderwand 6 hinausragt. Außerdem ist auf der Einrastplatte 22 eine Verschlusskante 24 vorgesehen. Die Verschlusskante 24 erstreckt sich über die Breite der Einrastplatte 22. Ebenfalls ist in der Figur 2 zu sehen, dass an die Verschlusskante 24 rampenartige Abschnitte 25 angrenzen, die eine Schräge bilden, um es der Kassette 1 zu erleichtern in das Gerät 3 einzurasten. Ebenfalls ist eine Schwenkachse 26 zu sehen, um welche die Kassette 1 in die Aufnahme 2 des Geräts 3 einschwenkbar ist und welche orthogonal zu einer vorbestimmten Schwenkrichtung 27 ausgerichtet ist.

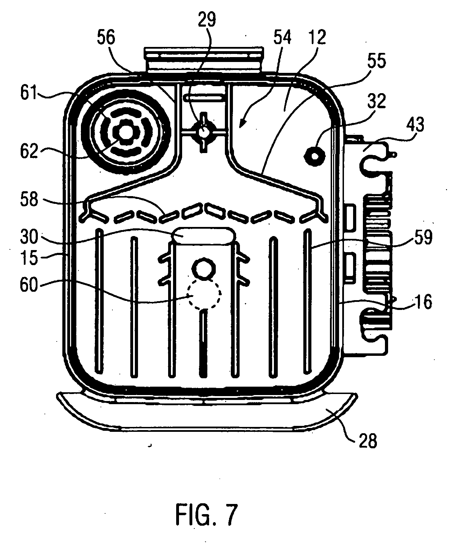

Auf der Rückseite 12 der Kassette 1 ist eine Öffnung 28 zur funktionalen Anordnung einer Vakuumpumpe des Geräts 3 vorgesehen. Die Öffnung 28 befindet sich an einem oberen rechten Bereich der Rückseite 12 und kann durch das Einschwenken der Kassette 1 in der vorbestimmten Schwenkrichtung 27 mit der im Gerät 3 vorgesehenen Pumpe funktionell verbunden werden. Außerdem ist neben der Öffnung 28 für die Pumpe eine weitere Öffnung 29 vorgesehen, die für eine Belüftung vorhanden ist. Die Öffnung 29 der Belüftung kann wahlweise durch einen nicht gezeigten Pneumatikzylinder als Steuerelement geschlossen bzw. geöffnet werden. Außerdem ist auf der Rückseite 12 der Kassette 1 in einem zentralen Bereich ein Fenster 30 ausgebildet. Durch das Fenster 30 kann ein in der Figur 2 nicht gezeigter Schwimmer 60 (siehe Figur 7) detektiert werden. Das Gehäuse 11 der Kassette 1 ist vakuumdicht, vorzugsweise aus Kunststoff gegossen und kann auch transparent ausgebildet sein.

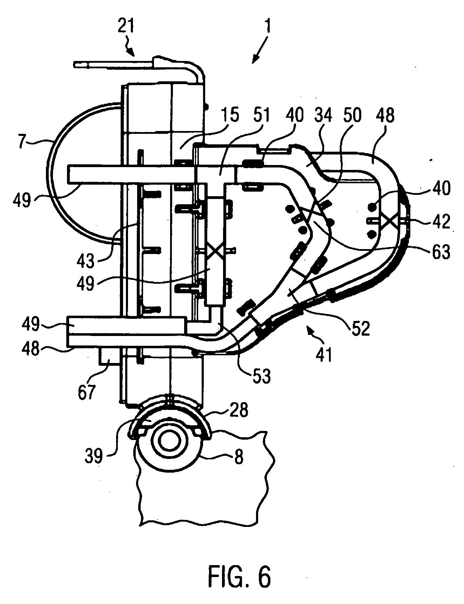

In einem linken oberen Bereich der Rückwand 12 der Kassette 1 ist ein Anschluss 32 ausgebildet, der für das Ansaugen von Aspirationsflüssigkeit vorhanden ist. Außerdem ist an der Seitenwand 15 ein Träger 33 vorhanden. Der Träger 33 dient zum Ausrichten von nicht gezeigten Fluidleitungen 41 (siehe Figur 6). Der Träger 33 umfasst eine Ausrichtplatte 34, die von der Seitenwand 15, insbesondere von einer Kante 31 der Seitenwand 15, absteht. Selbst wenn es schwer ersichtlich ist, ist die Ausrichtplatte 34 zur Schwenkrichtung 27 in einem Winkel zwischen 2 und 7 Grad geneigt. Auf der Ausrichtplatte 34 ist eine Abdeckung 35 angeordnet. Die Ausrichtplatte 34 und die Abdeckung 35 bilden zusammen ein Trägergehäuse 36. Die Ausrichtplatte 34 ist im Wesentlichen parallel zur Abdeckung 35 angeordnet. Am Rand der Abdeckung 35 ist ein Einrastglied 37 zu sehen, welches am Rand der Ausrichtplatte 34 in einer dafür vorgesehenen Einrastkerbe 37a eingerastet ist. Ebenfalls ist am Rand der Ausrichtplatte eine Umrandung 38 ausgebildet, mit der die Abdeckung 35 auf Abstand gehalten wird.

Die Figur 3 zeigt die erfindungsgemäße Kassette in einer anderen Perspektive. Zusätzlich zu sehen sind in der Lagerschale 18 Ausrichtungsrippen 39, die jeweils am ersten und am zweiten Ende 19, 20 im Führungsbereich 61 der Lagerschale 18 vorhanden sind. Die Ausrichtungsrippen 39 sind an die Form der Lagerkörper 8, wie sie in der Figur 1 gezeigt sind, angepasst. Insbesondere bei einer konischen Ausbildung der Lagerkörper 8 ist es mit den daran angepaßten Ausrichtungsrippen 39 möglich, die Kassette 1 so auf den Lagerkörpern 8 anzudocken, dass sich die Lagerschale 18 automatisch in die bevorzugte Schwenkrichtung 27 dreht, um die Kassette 1 in das Gerät einzuschwenken. Die Ausrichtungsrippen 39 sind zueinander parallel angeordnet, sodass sie ebenfalls parallel zur Schwenkrichtung 27 ausgebildet sind. Ebenfalls zeigt die Figur 3 eine Trennfüge 40, die die Vorderwand 6 mit dem Rest des Gehäuses 2 verbindet. Die Trennfuge 40 kann jedoch auch an anderer Stelle des Gehäuses 2 der Kassette 1 vorgesehen sein. Möglich ist es auch, dass mehrere Trennfugen 40 vorliegen, um unterschiedliche Abschnitte des Gehäuses 2 miteinander zu verbinden. Entlang der Trennfuge 40 kann ein Bindemittel, beispielsweise Klebstoff, vorgesehen sein, um die Vorderwand 6 auf dem übrigen Gehäuse 11 zu befestigen. Insbesondere kann ein Verbinden der Vorderwand 6 mit dem Rest des Gehäuses 2 entlang der Trennfuge 40 durch ein thermisches Schweißverfahren durchgeführt werden.

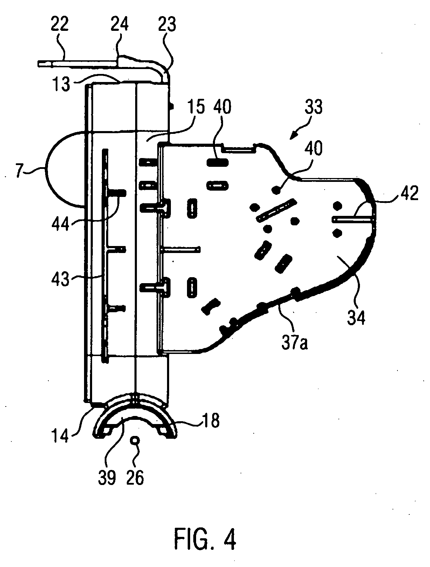

Die Figur 4 zeigt die erfindungsgemäße Kassette 1 aus der Seitenansicht. Zu sehen ist die Seitenwand 15, an der der Träger 33 hier ohne Abdeckung 35 befestigt ist. Auf der Ausrichtplatte 34 des Trägers 33 sind Halter 40 jeweils paarweise ausgebildet, die zur Halterung und Führung der Fluidleitungen 41 (siehe Figur 6) vorhanden sind. Die Halter 40 sind entweder als Platten oder als nadelförmige Stifte ausgebildet. Ebenfalls sind auf der Ausrichtplatte 34 Widerlagerkanten 42 vorhanden, die als Widerlager für die Fluidleitungen 41 dienen, wenn die Steuerelemente 63 auf diese wirken. Ebenfalls zu sehen in der Figur 4 ist die Lagerschale 18, die teilweise in dem Boden 14 der Kassette 1 eingesetzt eingeformt ist. Hier ist auch zu sehen, dass die Lagerschale 18 am Boden aus der Sicht des Betrachters nach rechts versetzt eingeformt ist. Ebenfalls sind die Ausrichtungsrippen 39 in der Lagerschale 18 zu sehen, welche an die Form der durch die Figur 1 gezeigten Lagerkörper 8 angepasst sind. Auf der Seitenwand 15 der Kassette 1 ist in der Figur 4 eine Führungsleiste 43 ausgebildet. Die Führungsleiste 43 steht im Wesentlichen senkrecht auf der Seitenwand 15. Ebenfalls erstrecken sich im Wesentlichen rechtwinklig von der Führungsleiste 43 Stützstreben 44. Um die Führungsleiste 43 stabil auf der Seitenwand 15 zu haltern.

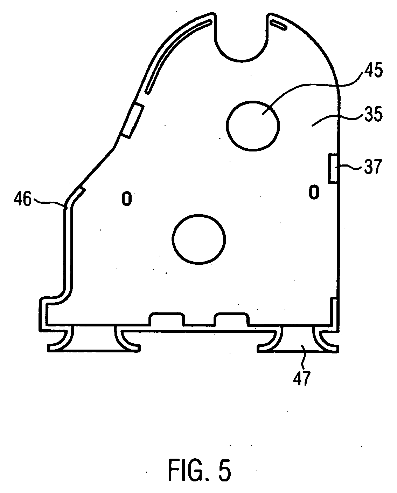

Die Figur 5 zeigt die Abdeckung 35 von der Ausrichtplatte 34 getrennt. In der Abdeckung 35 sind Betätigungsöffnungen 45 ausgebildet. Die Betätigungsöffnungen 45 sind deshalb in der Abdeckung 35 vorhanden, damit Steuerelemente 63 (siehe Figur 6) durch sie auf die Fluidleitungen 41 wirken können. Als Steuerelemente 63 sind beispielsweise Pneumatikzylinder vorgesehen, die im ausgefahrenen Zustand auf die Fluidleitungen 41 drücken, um diese abzuschließen bzw. im eingefahrenen Zustand einen Durchfluss in den Fluidleitungen ermöglichen. Entlang des Randes der Abdeckung 35 erstreckt sich teilweise eine Abdeckungsumrandung 46. Ebenfalls sind am Rand der Abdeckung 35 die bereits in der Figur 2 gezeigten Einrastglieder 37 zu sehen. Im unteren Rand der Abdeckung 35 sind in der Abdeckungsumrandung 46 zwei tulpenartige Öffnungen 47 ausgebildet. Die Öffnunggen 47 führen die Fluidleitungen 41 von der Kassette 1 fort. Die tulpenartigen Öffnungen 47 bieten ebenfalls einen Knickschutz für die Fluidleitungen 41.

In der Figur 6 ist die Anordnung der Fluidleitungen 41 an der Kassette 1 zu sehen. Die Fluidleitungen 41 weisen eine Aspirationsleitung 48, eine Infusionsleitung 49 sowie eine Backflushleitung 50 auf. Die Aspirationsleitung 48 ist entlang einem wesentlichen Abschnitt des Randes der Ausrichtplatte 34 geführt, verlässt in einem oberen Bereich der Ausrichtplatte 35 die Selbe und verläuft hinter dieser zu dem Anschluss 32 auf der Rückwand 12 der Kassette 1. Über die Infusionsleitung 49 wird das Auge mit Infusionsflüssigkeit versorgt, damit es nicht zusammenklafft. Die Backflushleitung 50 verbindet die Infusionsleitung 49 mit der Aspirationsleitung 48. Ebenfalls zeigt die Figur 6 einen T-förmigen Verbinder 51, der Abschnitte der Infusionsleitung 49 miteinander verbindet sowie die Infusionsleitung 49 fluidtechnisch mit einem ersten Ende der Backflushleitung 50 verbindet. Außerdem zeigt die Figur 6 einen Y-förmigen Verbinder 52, der ein zweites Ende der Backflushleitung 50 mit der Aspirationsleitung 48 sowie Abschnitte der Aspirationsleitung 48 miteinander verbindet. Die Halter 40 sorgen dafür, dass Abschnitte der Fluidleitungen 41 direkt über den Widerlagerkanten 42 angeordnet sind. Die Steuerelemente 63, die durch die Abdeckungsöffnungen 45 in das Trägergehäuse 36 einfahren, drücken auf die Fluidleitungen 41 um einen Fluidfluss zu unterbrechen. Schließlich sind in der Figur 6 die Lagerkörper 8 zu sehen, die in der Lagerschale 18 liegen und insbesondere an die Form der Ausrichtungsrippen 39 angepasst sind.

Die Figur 7 zeigt den inneren Aufbau der Kassette 1, wobei die Vorderwand 6 abgenommen ist. Auf der innigen Oberfläche der Rückwand 12 sind Lüfterrippen 54 ausgebildet. Die Lüfterrippen 54 umfassen eine erste und eine zweite Luftverteilerrippe 55; 56, die spiegelsymmetrisch geformt sind und zusammen im Wesentlichen einen nach unten gerichteten konischen Trichter bilden. Die Lüfterrippen 54 schließen die Öffnung 29 zur Belüftung ein und erstrecken sich vom Deckel 13 des Gehäuses 11 in Richtung des Bodens 14 der Kassette 1. Die erste und die zweite Luftverteilerrippe 55, 56 sind im Wesentlichen so geformt, dass sich der nach unten öffnende Trichter im Wesentlichen über die komplette Breite des Gehäuses 11 erstreckt. Über die breite Trichteröffnung erstrecken sich Dosierrippen 57, die im Wesentlichen dachartig, schräg gestellt angeordnet sind.

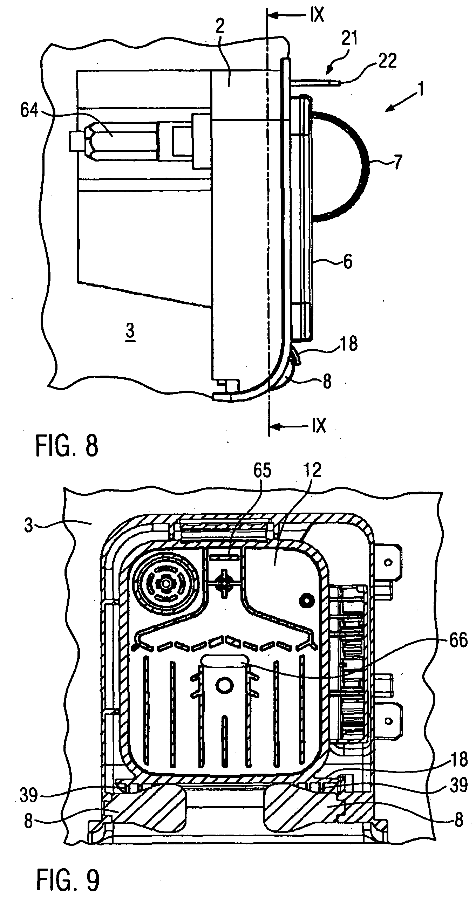

Unterhalb der Dosierrippen 57 sind parallel zu den Seitenwänden 15, 16 Strömungsrippen 59 vorhanden, die ein Schwappen des abgesaugten Aspirationsfluids in der Kassette 1 verhindern. Die Strömungsrippen 59 sind im Wesentlichen im gleichen Abstand zueinander angeordnet, wobei sie sich vorzugsweise nicht vollständig bis zum Boden 14 der Kassette 1 erstrecken. Dadurch ist es möglich, dass sich das abgesaugte Aspirationsfluid gleichmäßig über die Kassette 1 verteilt. Ebenfalls zu sehen ist ein Schwimmer 60, der mittig zwischen den Strömungsrippen 59 angeordnet ist. Sobald sich die Kassette 1 mit Aspirationsfluid füllt, wird der Schwimmer 60 angehoben und kann bei Erreichen einer vorbestimmten Höhe durch das Fenster 30 detektiert werden. Ebenfalls zeigt die Figur 7 die Öffnung 32, durch welche das Aspirationsfluid in die Kassette 1 strömt. In einem linken oberen Bereich der Rückwand 12 ist eine Filterhalterung 61 vorgesehen. Die Filterhalterung 61 trägt einen nicht gezeigten Filter, der lediglich durch Preßpassung in der Filterhalterung 61 haltbar ist. Die Figur 8 zeigt die Kassette 1 in der Aufnahme 2 aus seitlicher Ansicht. Ebenfalls zeigt die Figur 8 ein Belüftungssteuerelement 64, welches vorzugsweise als ein pneumatischer Zylinder vorgesehen ist. Das Belüftungssteuerelement 64 ist funktionell mit der für die Belüftung vorgesehenen Öffnung verbunden und kann durch eine nicht gezeigte, funktionell verbundene Fußschaltung betätigt werden. Die Kassette 1 wird durch die Verriegelung 21, insbesondere die Einrastplatte 22, mit ihrer Lagerschale 18 gegen die Lagerkörper 8 gespannt.

Schließlich zeigt die Figur 8 den Griff 7, welcher auf der Vorderseite 6 der Kassette 1 angeordnet. Nahe dem Deckel der Kassette 1 schließt der Griff 7 mit einem oberen Ende an die Vorderseite 6 der Kassette 1 an. Mit einem unteren Ende schließt der Griff 7 ungefähr in der Mitte der Vorderseite 6 der Kassette 1 an.

Wie bereits erwähnt wurde kann der Griff 7 als Lichtleiter ausgebildet sein, wobei er einen Lichtstrahl, der nahe dem Deckel der Kassette 1 eingeleitet wird, durch sein oberes Ende aufnimmt und an das untere Ende weiterleitet, um den Lichtstrahl an nicht gezeigte hinter der Rückseite der Kassette angeordnete Mittel zur Detektion des Lichts weiterzugeben. Dies ist möglich solange die zuvor erwähnte Schwimmerkugel 60 nicht durch den Füllstand der Kassette derart angehoben wurde, dass sie den Lichtstrahl durch die Kassette unterbricht.

Die Figur 9 zeigt einen vertikalen Schnitt entlang IX - IX, wie er durch die Figur 8 dargestellt ist. Durch die vertikale Schnittdarstellung der Figur 9 werden insbesondere die birnenförmigen Lagerkörper 8 gut sichtbar. Auf den birnenartigen Lagerkörpern 8 liegen die daran angepassten Ausrichtungsrippen 39 der Lagerschale 18 auf; um die Kassette 1 stabil zu lagern. In der Rückwand 12 der Kassette 1 ist ein erstes Fenster 65, sowie ein zweites Fenster 66 vorgesehen. Durch das erste Fenster 65 wird der zuvor erwähnte Lichtstrahl in das obere Ende des Griffs 7 eingeleitet. Der Griff 7 als Lichtleiter leitet den Lichtstrahl weiter zu seinem unteren Ende, welches zum zweiten Fenster 66 ausgerichtet ist. Falls die Schwimmerkugel 60 nicht bereits durch die Aspirationsflüssigkeit nach oben vor das zweite Fenster 66 gehoben wurde, wird der Lichtstrahl dort von Mitteln zur Lichtdetektion, die hinter der Kassette vorgesehen sind, empfangen. Dabei ist es möglich den Lichtstrahl kontinuierlich oder intervallweise durch den Griff 7 zu leiten. Ebenfalls ist vorgesehen, dass bei einem Ausbleiben der Detektion des Lichts ein Signal erfolgt, um dem Bediener mitzuteilen, dass die Schwimmerkugel bereits auf ein Niveau angehoben wurde, welches eine volle Kassette impliziert.

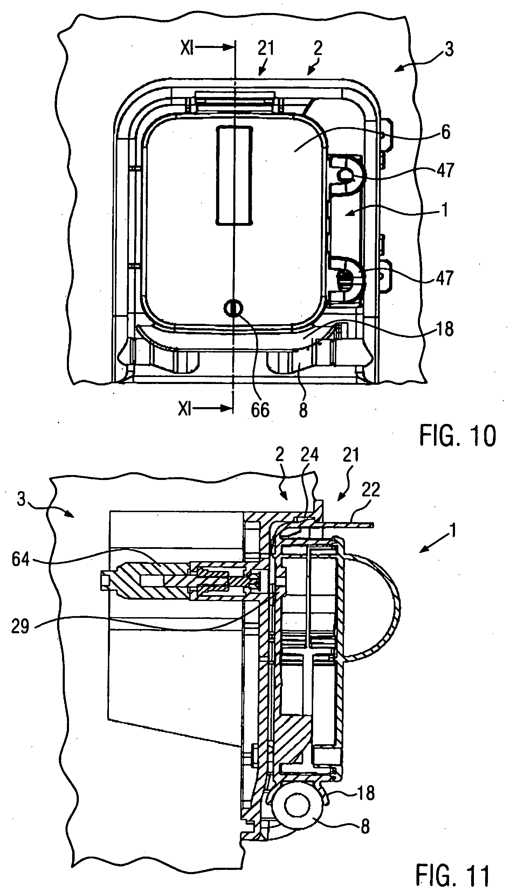

Die Figur 10 zeigt eine Vorderansicht der Kassette 1 in der Aufnahme 2 des Geräts 3. Die Kassette 1 ist in die Aufnahme 2 des Geräts eingeschwenkt und zwischen die Verriegelung 21 und die Lagerkörper 8 gespannt. Dabei liegt die Kassette 1 mit ihrer Lagerschale 18 auf den Lagerkörpern 8 auf. Die Lagerkörper 8 stehen von einer inneren Wandung der Aufnahme 2 nach innen gerichtet auf gleicher Höhe ab. Ebenfalls ist ein Anschluss 66 auf der Vorderwand 6 der Kassette 1 vorgesehen. Am Anschluss 66 der Vorderwand 6 kann ein nicht gezeigter Flüssigkeitssammelbeutel befestigt werden, um die durch das Vakuum angesaugte Aspirationsflüssigkeit aus der Kassette 1 aufzunehmen. Rechts sind ebenfalls die tulpenartigen Öffnungen 47 zu sehen, durch welche die Infusionsleitung 49 und die Aspirationsleitung 48 aus dem Trägergehäuse 36 geführt werden.

Die Figur 11 zeigt eine seitliche Schnittdarstellung entlang des Schnitts XI - XI (siehe Figur 10) der Kassette 1 in der Aufnahme 2 des Geräts 3. Die Kassette 1 sitzt mit ihrer Lagerschale 18 auf den Lagerkörpern 8 auf und wird durch die Verriegelung 21 in der Aufnahme 2 gehalten. Dabei ist die Verriegelung 21 mit der auf der Einrastplatte 22 vorgesehenen Verschlusskante 24 in einer Verriegelungsleiste des Geräts 3 eingerastet. Das Belüftungssteuerungselement 64 ist funktionell mit der Belüftungsöffnung 29 ausgerichtet. Zum Belüften der Kassette 1 kann die Öffnung 29 durch das Belüftungssteuerungselement 64 geöffnet werden, wodurch das Vakuum in der Kassette abgebaut wird und die bereits angesaugte Aspirationsflüssigkeit durch die Öffnung 66 in den Fluidsammelbeutel abläuft.

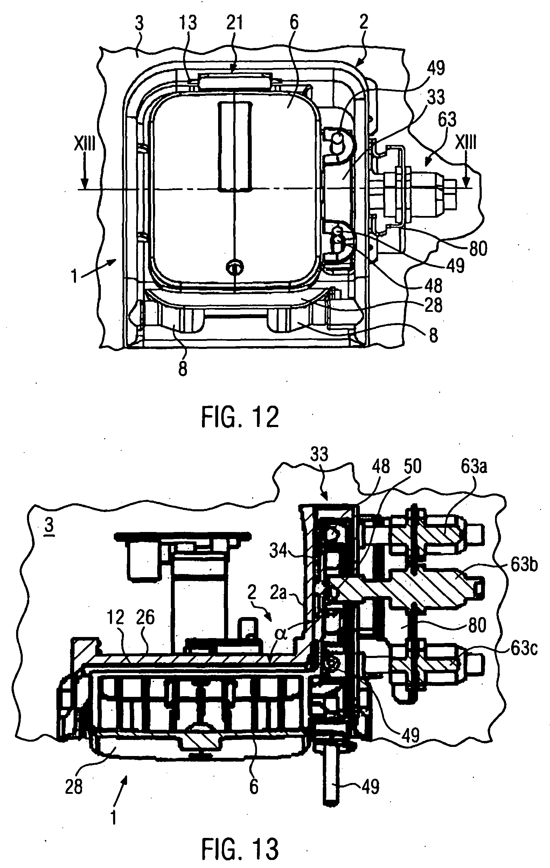

Die Figur 12 zeigt die Kassette 1 in der Aufnahme 2. Die Kassette 1 liegt mit ihrer Lagerschale 28 auf den birnenförmigen Lagerkörpern 8 auf und ist an ihrem Deckel 13 durch die Verriegelung 21 in der Aufnahme 2 befestigt. Der Träger 33 befindet sich derart in der Aufnahme 2, dass die Steuerelemente 63 funktionell zu ihm ausgerichtet sind. Die Steuerelemente 63 sind durch eine Halterung 80 an der Aufnahme 2 befestigt, damit sie relativ zur Aufnahme stabil gelagert sind. Außerdem ist in der Figur 12 ein Schnitt XIII - XIII gezeigt, dessen Ansicht anchsließend durch die Figur 13 erläutert wird.

Die Figur 13 zeigt den Schnitt XIII - XIII der Figur 12. Sie zeigt die Positionierung der Kassette 1 relativ zur Aufnahme 2 nachdem die Kassette 1 eingeschwenkt wurde. Die Rückwand 12 der Kassette 1 ist im Wesentlichen parallel zu einer Wandung 2b der Aufnahme 2 ausgerichtet. Die Ausrichtplatte 34 des Trägers 33 ist kraftschlüssig zu einer weiteren Wandung 2a der Aufnahme 2 ausgerichtet. Die Ausrichtplatte 34 des Trägers 33 schließt relativ zur Rückwand 12 der Kassette 1 einen Winkel α ein, der größer als 90° sein kann, vorzugsweise jedoch nicht größer als 97° ist. Durch die vorzugsweise relativ zur Rückwand 12 der Kassette 1 ausgestellte Ausrichtplatte ist es für den Benutzer einfacher den Träger 33 in die Aufnahme 2 ohne Ankanten einzuschwenken. Des Weiteren zeigt die Figur 13 die Steuerelemente 63 a, 63b, 63c, für Fluidleitungen die relativ zum Träger 33, insbesondere zur Ausrichtplatte 34, angeordnet sind. Die Steuerelemente 63a, 63b, 63c werden durch die Halterung 80 an der Aufnahme 2 befestigt. Dabei ist das Steuerelement 63a funktionell mit der Aspirationsleitung 48, das Steuerelement 63b funktionell mit der Backflashleitung 50 und das Steuerelement 63c funktionell mit der Infusionsleitung 49 ausgerichtet.

Durch das kraftschlüssige Anlegen der Ausrichtplatte 34 an der Wandung 2a der Aufnahme 2 ist es möglich, dass eine durch die Steuerelemente 63 ausgeübte Kraft durch die leicht schräg gestellte Wandung 2a absorbiert wird. Die vorzugsweise leichte Schrägstellung der Ausrichtplatte 34 hilft dem Benutzer auch dabei, wenn er die Kassette 1 aus der Aufnahme 2 ausschwenken möchte, weil die Ausrichtplatte 34 lediglich zu Beginn des Ausschwenkens an der Wandung 2a anliegt, jedoch dann von dieser freigegeben wird.

## Patentansprüche

1. Ophthalmologische Kassette (1) zum entfernbaren Anordnen in einer Aufnahme (2) eines ophthalmologischen Geräts (3), das Steuerelemente (63) für Fluidleitungen (41) aufweist, welche Kassette (1) ein Gehäuse (11) mit einer Vorderwand (6), einer Rückwand (12), einem Deckel (13), einem Boden (14) sowie mit Seitenwänden (15, 16) umfasst, wobei vornehmlich am Boden (14) ein Lager (10) für eine schwenkbare Anordnung der Kassette (1) am Gerät (2) vorhanden ist,
**dadurch gekennzeichnet,**
**dass** sich an einer der Seitenwände (15, 16) ein Träger (33) für die Fluidleitungen (41) befindet, der nach dem Einschwenken der Kassette (1) in das Gerät (2) die Steuerelemente (63) den Fluidleitungen (41) zuordnet.

2. Kassette nach Anspruch 1, **dadurch gekennzeichnet, dass** das Lager (10) mindestens eine Lagerschale (18) mit einem Führungsbereich (61) aufweist, wobei der Führungsbereich (61) zur Kopplung an mindestens einem, zumindest teilweise rotationssymetrischen Lagerkörper (8) ausgebildet ist.

3. Kassette nach Anspruch 2, **dadurch gekennzeichnet, dass** der Führungsbereich (61) zur Kopplung an einem zylindrischen oder konischen oder birnenförmigen oder ähnlichen rotationssymetrischen Lagerkörper (8) ausgebildet ist.

4. Kassette nach einem der vorigen Ansprüche, **dadurch gekennzeichnet, dass** insbesondere am Deckel (13) der Kassette (1) eine Verriegelung (21) ausgebildet ist, um die Kassette (1) nach dem Einschwenken am Gerät (3) lösbar zu sichern, wobei die Verriegelung (21) eine Einrastplatte (22) umfasst, die insbesondere im Wesentlichen parallel zum Deckel (13) angeordnet ist.

5. Kassette nach einem der vorigen Ansprüche, **dadurch gekennzeichnet, dass** vornehmlich in der Rückwand (12) der Kassette (1) Öffnungen (28, 29, 32) für eine dem Gerät (3) zugeordnete Pumpe, für eine Belüftung, sowie für eine Aspirationsleitung (48) vorhanden sind.

6. Kassette nach Anspruch 5, **dadurch gekennzeichnet, dass** nach dem Einschwenken der Kassette (1) in das Gerät (3) eine Fluidverbindung zwischen der Pumpe und der dafür vorgesehenen Öffnung (28) herstellbar ist.

7. Kassette nach einem der vorangehenden Ansprüche, **dadurch gekennzeichnet, dass** der Träger (33) für die Fluidleitungen (41) eine Ausrichtplatte (34) umfasst, die an einer der Seitenwände (15,16) ausgebildet ist, wobei die Ausrichtplatte (34) zur vorbestimmten Schwenkrichtung (27) eine Neigung zwischen 2° und 7° aufweist.

8. Kassette nach Anspruch 7, **dadurch gekennzeichnet, dass** auf der Ausrichtplatte (34) Halter (40) vorgesehen sind, durch die die Fluidleitungen (41) funktionstauglich vor den Steuerelementen (63) positionierbar sind, wobei die Halter (40) insbesondere senkrecht auf der Ausrichtplatte (34) stehen und/oder eine zylindrische nadelartige Form aufweisen und/oder in Form einer Platte ausgebildet sind.

9. Kassette nach Anspruch 7 oder 8, **dadurch gekennzeichnet, dass** an der Seitenwand (15, 16), die die Ausrichtplatte (34) trägt, eine Führungsleiste (43) für die Fluidleitungen (41) ausgebildet ist, wobei die Führungsleiste (43) senkrecht auf der Seitenwand (15, 16) ausgebildet ist.

10. Kassette nach Anspruch 9, **dadurch gekennzeichnet, dass** die Führungsleiste (43) Öffnungen umfasst, durch die die Fluidleitungen (41) führbar sind.

11. Kassette nach einem der vorigen Ansprüche, **dadurch gekennzeichnet, dass** die Fluidleitungen (41) eine Aspirationsleitung (48) für ein Aspirationsfluid, eine Infusionsleitung (49) für ein Infusionsfluid und/oder eine Backflushleitung (50), für eine eventuelle Okklusion in der Aspirationsleitung (48) aufweisen.

12. Kassette nach Anspruch 11, **dadurch gekennzeichnet, dass** die Aspirationsleitung (48) mit einer dafür vorgesehenen Öffnung (32) der Kassette (1) verbunden ist, um das Aspirationsfluid in die Kassette (1) zu leiten.

13. Kassette nach Anspruch 11 oder 12, **dadurch gekennzeichnet, dass** die Aspirationsleitung (48), die Infusionsleitung (49) sowie die Backflushleitung (50) durch jeweils ein zugeordnetes Steuerelement (63) betätigbar sind.

14. Kassette nach einem der vorigen Ansprüche, **dadurch gekennzeichnet, dass** die Fluidleitungen (41) im Wesentlichen orthogonal zur Schwenkrichtung (27) der Kassette (1) durch die Steuerelemente (63) betätigbar sind.

15. Kassette nach einem der vorigen Ansprüche, **dadurch gekennzeichnet, dass** in der Rückwand (12) ein Fenster (30) ausgebildet ist.

## Claims

1. An ophthalmologic cassette (1) for removable arrangement in an accommodation means (2) of an ophthalmologic device (3) having control elements (63) for fluid lines (41), said cassette (1) comprising a housing (11) with a front wall (6), a back wall (12), a cover (13), a base (14) as well as side walls (15, 16), wherein a bearing (10) for pivotably arranging the cassette (1) at the device (3) is provided preferably on the base (14),
**characterized in that**
one of the side walls (15, 16) has provided thereon a support (33) for the fluid lines (41), said support (33) associating the control elements (63) with the fluid lines (41), when the cassette (1) has been pivoted into the device (3).

2. The cassette according to claim 1, **characterized in that** the bearing (10) comprises at least one bearing shell (18) having a guiding area (61), wherein the guiding area (61) is configured for coupling to at least one, at least partially rotationally symmetrical bearing body (8).

3. The cassette according to claim 2, **characterized in that** the guiding area (61) is configured for coupling to a cylindrical or conical or pear-shaped or similarly shaped rotationally symmetrical bearing body (8).

4. The cassette according to one of the preceding claims, **characterized in that** in particular the cover (13) of the cassette (1) defines a locking mechanism (21) for securing the cassette (1) releasably in position at the device (3), when it has been pivoted thereinto, wherein the locking mechanism (21) comprises a snap-in plate (22) arranged in particular substantially parallel to the cover (13).

5. The cassette according to one of the preceding claims, **characterized in that** preferably the back wall (12) of the cassette (1) has provided therein openings (28, 29, 32) for a pump associated with the device (3), for ventilation, and for an aspiration line (48).

6. The cassette according to claim 5, **characterized in that**, after the cassette (1) has been pivoted into the device (3), a fluid connection can be established between the pump and the opening (28) provided therefor.

7. The cassette according to one of the preceding claims, **characterized in that** the support (33) for the fluid lines (41) comprises an orientation plate (34) formed on one of the side walls (15, 16), wherein the orientation plate (34) is inclined at an angle between 2° and 7° relative to the predetermined pivoting direction (27).

8. The cassette according to claim 7, **characterized in that** the orientation plate (34) has provided thereon holders (40) by means of which the fluid lines (41) are serviceably positionable in front of the control elements (63), wherein the holders (40) are especially positioned perpendicularly on the orientation plate (34) and/or have a cylindrical needle-like shape and/or are configured in the form of a plate.

9. The cassette according to claim 7 or 8, **characterized in that** the side wall (15, 16) carrying the orientation plate (34) has formed thereon a guide rail (43) for the fluid lines (41), wherein the guide rail (43) is formed perpendicularly on the side wall (15, 16).

10. The cassette according to claim 9, **characterized in that** the guide rail (43) comprises openings through which the fluid lines (41) can be guided.

11. The cassette according to one of the preceding claims, **characterized in that** the fluid lines (41) comprise an aspiration line (48) for an aspiration fluid, an infusion line (49) for the infusion fluid and/or a backflush line (50) for a possible occlusion in the aspiration line (48).

12. The cassette according to claim 11, **characterized in that** the aspiration line (48) communicates with an opening (32) provided in the cassette (1) for this purpose, so as to conduct the aspiration fluid into the cassette (1).

13. The cassette according to claim 11 or 12, **characterized in that** the aspiration line (48), the infusion line (49) and the backflush line (50) are operable by a respective control element (63) associated therewith.

14. The cassette according to one of the preceding claims, **characterized in that** the fluid lines (41) are operable by the control elements (63) substantially orthogonally to the pivoting direction (27) of the cassette (1).

15. The cassette according to one of the preceding claims, **characterized in that** a window (30) is formed in the back wall (12).

## Revendications

1. Cassette ophtalmologique (1) destinée à être agencée de manière amovible dans un logement d'accueil (2) d'un appareil ophtalmologique (3), qui présente des éléments de commande (63) pour des conduites de fluide (41), ladite cassette (1) comprenant un boitier (11) avec une paroi avant (6), une paroi arrière (12), un couvercle (13), un fond (14) ainsi que des parois latérales (15, 16), un palier (10) étant prévu principalement sur le fond (14) pour un agencement pivotant de la cassette (1) sur l'appareil (2),
**caractérisée**
**en ce que** sur l'une des parois latérales (15, 16) se trouve un support (33) pour les conduites de fluide (41), qui, après pivotement de la cassette (1) dans l'appareil (2), associe les éléments de commande (63) aux conduites de fluide (41).

2. Cassette selon la revendication 1, **caractérisée**
**en ce que** le palier (10) présente au moins une coque de palier (18) avec une zone de guidage (61), la zone de guidage (61) étant configurée pour le couplage à un corps de palier (8) au moins partiellement à symétrie de rotation.

3. Cassette selon la revendication 2, **caractérisée**
**en ce que** la zone de guidage (61) est configurée pour le couplage à un corps de palier (8) à symétrie de rotation de forme cylindrique ou conique, ou en forme de poire, ou de forme similaire.

4. Cassette selon l'une des revendications précédentes, **caractérisée en ce que** notamment sur le couvercle (13) de la cassette (1) est formé un verrouillage (21) pour sécuriser la cassette (1) de manière démontable sur l'appareil (3) après l'y avoir fait pivoter, le verrouillage (21) comprenant une plaque d'encliquetage (22) qui est en particulier agencée sensiblement de manière parallèle au couvercle (13).

5. Cassette selon l'une des revendications précédentes, **caractérisée en ce que** principalement dans la paroi arrière (12) de la cassette (1) existent des ouvertures (28, 29, 32) pour une pompe associée à l'appareil (3), pour une ventilation, ainsi que pour une conduite d'aspiration (48).

6. Cassette selon la revendication 5, **caractérisée**
**en ce qu'**après le pivotement de la cassette (1) dans l'appareil (3), une liaison fluidique peut être établie entre la pompe et l'ouverture (28) prévue à cet effet.

7. Cassette selon l'une des revendications précédentes, **caractérisée en ce que** le support (33) pour les conduites de fluide (41) comprend une plaque d'orientation (34), qui est formée sur l'une des parois latérales (15, 16), la plaque d'orientation (34) présentant une inclinaison entre 2° et 7° par rapport à la direction de pivotement (27) prédéterminée.

8. Cassette selon la revendication 7, **caractérisée**
**en ce que** sur la plaque d'orientation (34) sont prévus des éléments de maintien (40) par lesquels les conduites de fluide (41) peuvent être positionnées, de manière apte sur le plan fonctionnel, devant les éléments de commande (63), les éléments de maintien (40) étant notamment agencés sur la plaque d'orientation (34) de manière normale à celle-ci, et/ou présentant une forme d'aiguille cylindrique et/ou étant réalisés sous forme de plaque.

9. Cassette selon la revendication 7 ou la revendication 8, **caractérisée en ce que** sur la paroi latérale (15, 16), qui porte la plaque d'orientation (34), est formée une barre de guidage (43) pour les conduites de fluide (41), la barre de guidage (43) étant réalisée sur la paroi latérale (15, 16) de manière normale à celle-ci.

10. Cassette selon la revendication 9, **caractérisée**
**en ce que** la barre de guidage (43) présente des ouvertures à travers lesquelles il est possible de faire passer les conduites de fluide (41).

11. Cassette selon l'une des revendications précédentes, **caractérisée en ce que** les conduites de fluide (41) comprennent une conduite d'aspiration (48) pour un fluide d'aspiration, une conduite d'infusion (49) pour un fluide d'infusion et/ou une conduite de décolmatage ou de circulation inversée (50) pour une éventuelle occlusion dans la conduite d'aspiration (48).

12. Cassette selon la revendication 11,
**caractérisée en ce que** la conduite d'aspiration (48) est reliée à une ouverture (32) de la cassette (1), prévue à cet effet, pour diriger le fluide d'aspiration dans la cassette (1) .

13. Cassette selon la revendication 11 ou la revendication 12, **caractérisée en ce que** la conduite d'aspiration (48), la conduite d'infusion (49) ainsi que la conduite de décolmatage (50) peuvent être actionnées chacune par un élément de commande (63) respectivement associé.

14. Cassette selon l'une des revendications précédentes, **caractérisée en ce que** les conduites de fluide (41) peuvent être actionnées par les éléments de commande (63), sensiblement de manière orthogonale à la direction de pivotement (27) de la cassette (1).

15. Cassette selon l'une des revendications précédentes, **caractérisée en ce que** dans la paroi arrière (12) est formée une fenêtre (30).
